# EUROPÄISCHE PATENTSCHRIFT

(11) **EP 1 081 126 B1**
(45) Veröffentlichungstag und Bekanntmachung des Hinweises auf die Patenterteilung: **05.03.2008**
(21) Anmeldenummer: 00117875.5
(22) Anmeldetag: 19.08.2000
(51) Int. Cl.: C07C 69/28, C07C 69/75, C07C 67/08

(54) **Verfahren zur Herstellung von Esterweichmachern**
Process for the preparation of plasticiser esters
Procédé de préparation d'esters plastifiants

(30) Priorität: 28.08.1999 DE 19940991
(43) Veröffentlichungstag der Anmeldung: 07.03.2001
(73) Patentinhaber: Celanese Chemicals Europe GmbH, 60439 Frankfurt am Main (DE)
(72) Erfinder: Springer, Helmut, 46539 Dinslaken (DE); Merscher, Klaus-Dieter, 64572 Büttelborn (DE); Heumüller, Rudolf, Dr., Unit 10B, Celestial Garden, Hong Kong (CN); Schimmer, Klaus, 69221 Dossenheim (DE); Strutz, Heinz, Dr., 61250 Usingen (DE)

(56) Entgegenhaltungen:
- EP-A- 0 024 722
- US-A- 2 229 222
- CHEMICAL ABSTRACTS, vol. 49, no. 10, 25. Mai 1955 (1955-05-25) Columbus, Ohio, US; Y. ISHII: "Syntheses of plasticizers. XII. Synthesis and plasticizing properties of polyalkyleneglycol plasticizers" Spalte 6904; XP002188592 & J. CHEM. SOC. JAPAN, Bd. 56, 1953, Seiten 945-949,
- DATABASE CROSSFIRE BEILSTEIN [Online] Beilstein Institut zur Förderung der chemischen Wissenschaften, Frankfurt am Main, DE; Database-Accession no. 888888 (BRN 1785345) , XP002188628 & JUSTUS LIEBIGS ANN. CHEM., Bd. 114, 1860, Seite 126

## Beschreibung

Die Erfindung betrifft ein Verfahren zur Herstellung von Esterweichmachern aus Ethylenglykol oder dem Di-, Tri- oder Tetraoligomeren dieser Grundverbindung und linearen oder verzweigten aliphatischen Monocarbonsäuren mit 3 bis 20 Kohlenstoffatomen in Gegenwart von organischen Substanzen mit einem Siedepunkt <112°C, die mit Wasser Azeotrope bilden (im folgenden auch als Azeotropbildner oder Schleppmittel bezeichnet) zur Entfernung des Reaktionswassers, wobei das Schleppmittel dem Reaktionsgemisch bei Erreichen einer Temperatur von mindestens 140°C hinzugefügt wird.

Weichmacher finden in großem Umfang und in vielfältiger Weise Anwendung in Kunststoffen, Beschichtungsmitteln, Dichtungsmassen, Kautschuk- und Gummiartikeln. Sie treten mit hochpolymeren thermoplastischen Stoffen, ohne chemisch zu reagieren, vorzugsweise durch ihre Löse- und Quellvermögen in physikalische Wechselwirkung. Hierdurch bildet sich ein homogenes System aus, dessen thermoplastischer Bereich gegenüber den ursprünglichen Polymeren zu niederen Temperaturen verschoben ist, u.a. mit dem Ergebnis, daß seine mechanischen Eigenschaften optimiert, z.B. Formveränderungsvermögen, Elastizität, Festigkeit erhöht werden und die Härte verringert wird.

Um Weichmachern möglichst weite Anwendungsgebiete zu eröffnen, müssen sie eine Reihe von Kriterien erfüllen. Im Idealfall sollten sie geruchlos, farblos, licht-, kälte- und wärmebeständig sein. Überdies erwartet man, daß sie unempfindlich gegenüber Wasser, schwer brennbar und wenig flüchtig sind und die Gesundheit nicht schädigen. Weiterhin soll die Herstellung der Weichmacher einfach sein und, um ökologischen Anforderungen zu genügen, unter Vermeidung von Abfallstoffen, wie nicht weiterverwertbaren Nebenprodukten und Schadstoffe enthaltenden Abwässern, erfolgen.

Zu den wichtigsten Weichmachern gehören die Ester von Di- und Polycarbonsäuren mit Weichmacheralkoholen, d.h. unverzweigten oder verzweigten primären Alkoholen mit etwa 6 bis 20 Kohlenstoffatomen, die als individuelle Verbindungen oder auch als Gemische eingesetzt werden. Die Herstellung der Ester erfolgt nach dem klassischen Verfahren durch Umsetzung der Säuren oder Säureanhydride mit einem Alkohol oder einem Gemisch unterschiedlicher Alkohole in Gegenwart einer Säure, vorzugsweise Schwefelsäure als Katalysator.

Eine spezielle Klasse von Esterweichmachern (sie werden kurz als G-Ester bezeichnet) enthält als Alkoholkomponente Diole bzw. Etherdiole, nämlich Ethylenglykol, Diethylenglykol, Triethylenglykol und Tetraethylenglykol. Ihre Herstellung kann auf unterschiedlichen Wegen erfolgen. Neben der Umsetzung von Alkohol und Säure gegebenenfalls in Gegenwart saurer Katalysatoren werden in der Praxis noch weitere Prozesse zur Gewinnung von G-Estern angewandt, u.a. die Reaktion von Diol mit Säurehalogenid, die Umesterung eines Carbonsäureesters mit einem Diol und die Addition von Ethylenoxid an Carbonsäuren (Ethoxylierung). In der industriellen Fertigung haben sich lediglich die unmittelbare Umsetzung von Diol und Carbonsäure und die Ethoxylierung von Carbonsäuren als Produktionsverfahren durchgesetzt, wobei der Veresterung von Diol und Säure zumeist der Vorzug gegeben wird. Denn dieses Verfahren kann ohne besonderen Aufwand in konventionellen chemischen Apparaten durchgeführt werden und es liefert chemisch einheitliche Produkte. Demgegenüber erfordert die Ethoxylierung umfangreiche und kostenintensive technische Mittel. Ethylenoxid ist eine sehr aggressive chemische Substanz. Es kann explosionsartig polymerisieren und bildet mit Luft in sehr weiten Mischungsbereichen explosive Gemische. Ethylenoxid reizt die Augen und Atemwege, führt zu Verätzungen, zu Leber- und Nierenschäden und ist karzinogen. Seine Handhabung erfordert daher umfangreiche Sicherheitsmaßnahmen. Überdies ist auf peinliche Sauberkeit von Lagervorrichtungen und Reaktionsapparaten zu achten, um die Bildung unerwünschter Verunreinigungen durch Nebenreaktionen des Ethylenoxids mit Fremdstoffen auszuschließen. Schließlich ist die Reaktion mit Ethylenoxid nicht sehr selektiv, denn sie führt zu Gemischen von Verbindungen unterschiedlicher Kettenlänge.

Die unmittelbare Veresterung von Alkoholen mit Carbonsäuren gehört zu den Grundoperationen der organischen Chemie. Um die Reaktionsgeschwindigkeit zu erhöhen, führt man die Umsetzung üblicherweise in Gegenwart von Katalysatoren durch. Der Einsatz eines der Reaktanten im Überschuß und/oder die Abtrennung des im Verlauf der Reaktion gebildeten Wassers stellt sicher, daß das Gleichgewicht entsprechend dem Massenwirkungsgesetz zur Seite des Reaktionsproduktes, also des Esters, verschoben wird, d.h. hohe Ausbeuten erzielt werden.

Wegen der eingangs beschriebenen Qualitätskriterien für Esterweichmacher sind die Auswahl des Katalysators und die Vorgehensweise bei der Abtrennung des Reaktionswassers sehr wesentliche Prozeßmerkmale. Denn beide Verfahrenseigenheiten beeinflussen in erheblichem Maße sensorische und optische Eigenschaften der Endprodukte. Die Struktur der Ausgangsstoffe, Alkohol und Säure, ist dagegen für die mechanischen und thermischen Eigenschaften der Weichmacher maßgebend.

Obgleich sich Geruch und Farbe der Weichmacher durch Zusatz von Additiven den gewünschten Anforderungen anpassen lassen, vermeidet man die Verwendung von Hilfsstoffen, weil sie andere Eigenschaften der Weichmacher beeinträchtigen können und/oder ihre Einsatzmöglichkeiten, z.B. wegen Unverträglichkeit mit dem Substrat, begrenzen.

Für die Abtrennung des bei der Esterbildung aus Ethylenglykol (und dessen Oligomeren) und Carbonsäuren gebildeten Reaktionswassers sind verschiedene Verfahren bekannt. Anwendung findet vorzugsweise die Azeotropdestillation in Gegenwart eines mit Wasser nicht mischbaren Lösemittels, das Erhitzen des Reaktionsgemisches unter Durchleiten eines inerten Gases, die Umsetzung der Ausgangsstoffe Alkohol und Carbonsäure unter Vakuum oder in Gegenwart eines Trocknungsmittels.

Aus US-A-2,229,222 ist die Herstellung von Diestern der Hexansäuren, beispielsweise n-Hexansäure oder 2-Ethylbuttersäure, und Polyglykolen, beispielsweise Di-, Tri- oder Tetraethylenglykol bekannt. Die Veresterungsreaktion erfolgt bei Säureüberschuß in Gegenwart eines sauren Katalysators und eines Azeotropbildners zur Wasserentfemung, beispielsweise Toluol oder Benzol, wobei der gesamte Reaktionsansatz vorgelegt und aufgeheizt wird. Die bekannten Esterverbindungen eignen sich als Weichmacher für Kunststoffe.

Gegenstand der EP-A1-0 024 722 ist die Verbindung Tetraethylenglykoldiheptanoat. Der Ester wird durch Erhitzen von Diol und Carbonsäure im Molverhältnis von 2,2 zu 1 in Gegenwart eines Gemisches aus Ameisensäure, Schwefelsäure als Katalysator hergestellt. Das als Schleppmittel verwendete Toluol wird dem Reaktionsgemisch zu Beginn der Aufheizphase zugesetzt.

Chem. Abs., Band 49, Nr. 10, Spalte 6904 betrifft die Herstellung von Diestern aus Glykolen, beispielsweise die Veresterung von Tetraethylenglykol mit n-Hexansäure. Die Veresterung erfolgt durch Aufheizen des gesamten Reaktionsansatzes in Gegenwart von para-Toluolsulfonsäure als Katalysator und von Xylol als Azeotropbildner. Die bekannten Esterverbindungen eignen sich als Weichmacher für PVC.

Aus Database Crossfire Beilstein, Beilstein Reg. Nr. 1,785,345 ist der Diester aus Monoethylenglykol und 3-Methylbuttersäure bekannt.

Insbesondere die Wasserentfernung durch azeotrope Destillation hat sich für die Einstellung des Gleichgewichts bei der Herstellung von Esterweichmachern bewährt. Dennoch stellen die bekannten Verfahren und die bisher eingesetzten Schleppmittel nicht sicher, daß die hohen, für Weichmacher geforderten Qualitätsstandards, erzielt werden.

Aufgabe der vorliegenden Erfindung ist es daher ein Verfahren bereitzustellen, das die Herstellung von Weichmacherestern auf Basis von Ethylenglykol und oligomeren Ethylenglykolen hoher Reinheit und in hohen Ausbeuten erlaubt. Besonderer Wert wird in diesem Zusammenhang darauf gelegt, daß der Prozeß mit einfachen technischen Mitteln realisiert werden kann, lange Betriebszeiten sicherstellt und über die Betriebsdauer gleichbleibende Produkte einwandfreier Qualität liefert.

Die Erfindung besteht in einem Verfahren zur Herstellung von Esterweichmachern durch Umsetzung von Mono-, Di-, Tri- oder Tetraethylenglykolen mit linearen oder verzweigten aliphatischen Monocarbonsäuren mit 3 bis 20 Kohlenstoffatomen in Gegenwart eines Schleppmittels zur Entfernung des im Verlauf der Umsetzung gebildeten Wassers als azeotropes Gemisch. Das Verfahren ist dadurch gekennzeichnet, daß als Schleppmittel Substanzen aus der breffe bestehend aus Hexan, 1-Hexan, Cyclohexan und Toluol verwendet werden, und dass daß Schleppmittel dem Reaktionsgemisch bei Erreichen einer Temperatur von mindestens 140°C, insbesondere 150 bis 170°C, hinzugefügt wird.

Die neue Arbeitsweise zeichnet sich durch große Zuverlässigkeit nicht nur im Labor- und Versuchsbetrieb, sondern vor allem auch in technischen Anlagen aus. Sie ist, auch kontinuierlich, leicht durchzuführen und liefert Weichmacher hoher Reinheit. Besonders bemerkenswert ist die problemlose und vollständige Abtrennung des Reaktionswassers und auch des zur Entfernung des Wassers verwendeten Schleppmittels. Die restlose Abtrennung des Reaktionsnebenproduktes und des Hilfsstoffes hat die ausgezeichneten Farbeigenschaften und die bemerkenswerte Farbstabilität der Esterweichmacher zur Folge.

Ein entscheidendes Merkmal des erfindungsgemäßen Verfahrens ist die Entfernung des Reaktionswassers aus dem Reaktionsgemisch und damit die Verschiebung des Gleichgewichtes zu Gunsten des Esters mit Hilfe von Hexan, 1-Hexen, Cyclohexan oder Toluol. Als besonders vorteilhafter Azeotropbilder hat sich Cyclohexan erwiesen. Cyclohexan bildet mit Wasser ein niedrig siedendes binäres System, das sich leicht aus dem Gemisch der Reaktanten und des Produktes abdestillieren läßt. Das Auftreten binärer oder ternärer Gemische mit den Reaktionspartnern und dem Ester wird nicht beobachtet. Auch die Entfernung überschüssigen Cyclohexans aus dem Reaktionsgemisch bereitet auf Grund seines niedrigen Siedepunkts keine Schwierigkeiten. Die chemische Stabilität und die Indifferenz des cycloaliphatischen Kohlenwasserstoffs stellt sicher, daß das Reaktionsprodukt durch Umwandlungsprodukte nicht verunreinigt wird.

Die zur vollständigen Abtrennung des Wassers erforderliche Menge Schleppmittel läßt sich aus der entsprechend der Stöchiometrie der Veresterungsreaktion berechneten Wasserbildung und aus der Zusammensetzung des binären Azeotrops in einfacher Weise ermitteln. Es hat sich bewährt, das Schleppmittel im Überschuß einzusetzen, zweckmäßig in einem Anteil, der 50 bis 200 Gew.-% über der theoretisch berechneten Menge liegt.

Nach der erfindungsgemäßen Arbeitsweise setzt man den Azeotropbildner dem Reaktionsgemisch erst bei Erreichen einer Temperatur von mindestens 140°C, insbesondere 150 bis 170°C, zu und nicht vor oder während des Aufheizvorganges. Diese Maßnahme führt zu einer besonders schonenden und wirksamen Abtrennung des Wassers. Das Schleppmittel kann dem Reaktionsgemisch in Anteilen oder vorteilhaft kontinuierlich in dem Maße hinzugefügt werden, wie es durch Azeotropbildung verbraucht wird. Durch Auffangen und Auftrennen des abdestillierten Schleppmittel-/Wassergemischs läßt sich in einfacher Weise der Fortgang der Reaktion verfolgen. Das aus dem Azeotrop abgeschiedene Schleppmittel kann unmittelbar, d.h. ohne die Zwischenschaltung einer Reinigungsstufe, in die Reaktion zurückgeführt werden.

Die als Ausgangsstoffe für das erfindungsgemäße Verfahren eingesetzten Mono-, Di-, Tri- und Tetraethylenglykole sind industriell produzierte Chemikalien. Basissubstanz zu ihrer Herstellung ist Ethylenoxid, aus dem man (Mono-) Ethylenglykol durch Erhitzen mit Wasser unter Druck gewinnt. Diethylenglykol erhält man durch Ethoxylierung aus Ethylenglykol. Triethylenglykol fällt, wie Tetraethylenglykol, als Nebenprodukt bei der Hydrolyse von Ethylenoxid zur Herstellung von Ethylenglykol an. Beide Verbindungen können auch durch Umsetzung von Ethylenglykol mit Ethylenoxid synthetisiert werden.

Zur Gewinnung von Estern nach dem erfindungsgemäßen Prozeß setzt man lineare oder verzweigte, aliphatische Monocarbonsäuren mit 3 bis 20 Kohlenstoffatomen im Molekül ein. Obgleich man gesättigten Säuren in vielen Fällen den Vorzug gibt, können, in Abhängigkeit vom jeweiligen Anwendungsgebiet der Weichmacher, auch ungesättigte Carbonsäuren als Reaktionskomponente zur Estersynthese verwendet werden. Beispiele für Carbonsäuren als Bausteine von G-Estern sind n-Buttersäure, Isobuttersäure, n-Pentansäure, 2-Methylbuttersäure, 3-Methylbuttersäure, 2-Methylpentansäure, 2-Ethylbuttersäure, n-Heptansäure, 2-Methylhexansäure, Cyclohexancarbonsäure, 2-Ethylhexansäure, n-Nonansäure, 2-Methyloctansäure, Isononansäure, 2-Methylundecansäure, Isoundecancarbonsäure, Tricyclodecancarbonsäure und Isotridecancarbonsäure. Besonders bewährt hat sich das neue Verfahren für die Herstellung von Estern des Monoglykols bzw. der oligomeren Glykole mit C₄- bis C₁₃- vzw. C₅- bis C₉-Monocarbonsäuren

Die Reaktion von Glykolen und Carbonsäuren kann ohne Einsatz eines Katalysators durchgeführt werden. Diese Variante der Umsetzung hat den Vorteil, daß man vermeidet, dem Reaktionsgemisch Fremdstoffe zuzuführen, die zu einer unerwünschten Verunreinigung des Esters führen kann. Allerdings muß man dann im allgemeinen höhere Reaktionstemperaturen einhalten, weil nur so gewährleistet ist, daß die Umsetzung mit ausreichender, d.h. wirtschaftlich vertretbarer Geschwindigkeit abläuft. Zu beachten ist in diesem Zusammenhang, daß die Steigerung der Temperatur zu einer thermischen Schädigung des Esters führen kann. Daher läßt sich nicht immer die Verwendung eines Katalysators, der die Umsetzung erleichtert und die Reaktionsgeschwindigkeit erhöht, vermeiden. Häufig kann der Katalysator ein Überschuß der Säure sein, die gleichzeitig Reaktionskomponente des Glykols ist, Im übrigen sind die üblichen Veresterungskatalysatoren zur Beeinflussung der Reaktionsgeschwindkeit geeignet, wie Schwefelsäure, Ameisensäure, Polyphosphorsäure, Methansulfonsäure oder p-Toluolsulfonsäure und ebenso Kombinationen derartiger Säuren. Bevorzugt werden unter Reaktionsbedingungen feste, im Reaktionssystem unlösliche, katalytisch wirksame Verbindungen wie Alkali- oder Erdalkalihydrogensulfat, insbesondere Natriumhydrogensulfat, eingesetzt, da diese nach Beendigung der Veresterung durch einfache Filtration aus dem Reaktionsgemisch entfernt werden können und anschließend keine zusätzliche Behandlung der Reaktionsmischung notwendig wird. Die Menge des eingesetzten Katalysators kann sich über einen weiten Bereich erstrecken. Es können sowohl 0,01 Gew.-% als auch 5 Gew.-% Katalysator, bezogen auf das Reaktionsgemisch, verwendet werden. Da größere Katalysatormengen jedoch kaum Vorteile ergeben, beträgt die Katalysatorkonzentration üblicherweise 0,01 bis 1,0, vorzugsweise 0,01 bis 0,5 Gew.-%, jeweils bezogen auf das Reaktionsgemisch. Zweckmäßig entscheidet man gegebenenfalls durch Vorversuche für jeden Einzelfall, ob ohne Katalysator bei höherer Temperatur oder mit Katalysator bei niedrigerer Temperatur zu arbeiten ist.

Die Veresterung kann mit stöchiometrischen Mengen Alkohol und Säure vorgenommen werden. Vorzugsweise läßt man das Diol jedoch mit überschüssiger Säure reagieren, um in endlicher Zeit eine möglichst vollständige Umsetzung zu erreichen.

Die Reaktion zwischen Alkohol und Säure setzt in Abhängigkeit von den Einsatzmaterialien im Bereich von etwa 150 bis 170°C ein. Sie kann bei Temperaturen bis zu etwa 250°C zu Ende geführt werden. Bei diesen Temperaturangaben handelt es sich um Richtwerte, die zweckmäßig eingehalten werden. Niedrigere Temperaturen können z.B. ausreichen, wenn im speziellen Fall eine genügend hohe Reaktionsgeschwindigkeit erzielt wird oder nur Teilumsätze angestrebt werden. Höhere Temperaturen sind möglich, wenn das Auftreten von Zersetzungsprodukten, die u.a. farbschädigend wirken, auszuschließen ist. Die Anwendung von vermindertem oder erhöhtem Druck ist nicht ausgeschlossen, sie wird sich jedoch auf Sonderfälle beschränken.

Das nach Beendigung der Umsetzung anfallende Reaktionsgemisch enthält neben dem Ester als erwünschtem Reaktionsprodukt gegebenenfalls nichtumgesetzte Einsatzstoffe, insbesondere noch überschüssige Säure (sofern mit einem Säureüberschuß gearbeitet wurde). Zur Aufarbeitung wird der Reaktoraustrag nach konventionellen Verfahren vom Katalysator befreit. Liegt der Katalysator als Feststoff vor, z.B. in Form eines Hydrogensulfats, filtriert man das Produkt in üblichen Filtrierapparaten bei normaler Temperatur oder bei Temperaturen bis 150°C. Die Filtration kann durch gängige Filterhilfsmittel, wie Zellulose, Kieselgel, Kieselgur, Holzmehl, unterstützt werden. Anschließend destilliert man überschüssige und nichtumgesetzte Ausgangsstoffe ab. Um letzte Reste acider Bestandteile zu entfernen, kann auch noch eine Behandlung mit einem alkalischen Reagenz, z.B. wäßriger Soda- oder Natriumhydroxidlösung, vorgesehen werden. Nach Phasentrennung wird der Ester getrocknet, beispielsweise, indem man ein inertes Gas durch das Produkt leitet oder Vakuum anlegt. Sofern der Katalysator im Reaktionsgemisch gelöst ist, wie Schwefelsäure oder p-Toluolsulfonsäure, destilliert man gegebenenfalls nach vorangegangener Filtration, zunächst noch vorhandene Ausgangsstoffe ab, behandelt darauf mit einem alkalischen Reagenz und trocknet das Produkt.

Falls es der vorgesehene Verwendungszweck erfordert, können sich der Isolierung des Esters noch weitere Reinigungsschritte anschließen, beispielsweise eine Destillation im Vakuum.

Nach dem neuen Verfahren wurden eine Reihe Diester des Mono-, Di-, Tri- und Tetraethylengykols hergestellt, Im einzelnen handelt es sich um die Diester des Monoethylenglykols mit jeweils einer der folgenden Carbonsäuren: 3-Methylbuttersäure, 2-Methylhexansäure, 2-Methyloctansäure, 3,5,5-Trimethylhexansäure, insbesondere in Form von Isononansäure, einem technischen Produkt, das etwa 95 Gew.-% 3,5,5-Trimethylhexansäure enthält, 2-Methylundecansäure; um die Diester des Diethylenglykols mit jeweils einer der folgenden Carbonsäuren: 2-Methylbuttersäure, 3-Methylbuttersäure, 2-Methyloctansäure, 2-Methylundecansäure; um die Diester des Triethylenglykols mit jeweils einer der folgenden Carbonsäuren: 2-Methylbuttersäure, 2-Methylhexansäure, Cyclohexancarbonsäure, 2-Methyloctansäure, 3,5,5-Trimethylhexansäure, insbesondere in Form von Isononansäure, einem technischen Produkt, das etwa 95 Gew.-% 3,5,5-Trimethylhexansäure enthält, 2-Methylundecansäure; schließlich um die Diester des Tetraethylenglykols mit jeweils einer der folgenden Carbonsäuren: Isobuttersäure, n-Pentansäure, 2-Methylbuttersäure, 3-Methylbuttersäure, n-Hexansäure, 2-Methylpentansäure, 2-Methylhexansäure, Cyclohexancarbonsäure, 2-Methyloctansäure, 3,5,5-Trimethylhexansäure, insbesondere in Form von Isononansäure, einem technischen Produkt, das etwa 95 Gew.-% 3,5,5-Trimethylhexansäure enthält und 2-Methylundecansäure.

Die Ester des Ethylenglykols sowie seine Oligomeren eignen sich hervorragend als Weichmacher für alle gängigen hochpolymeren thermoplastischen Stoffe. Besonders bewährt haben sie sich als Zusatz zu Polyvinylbutyral, das mit Glykolestern versetzt als Zwischenschicht zur Herstellung von Mehrschichten- oder Verbundgläsern verwendet wird.

Das erfindungsgemäße Verfahren kann absatzweise oder auch kontinuierlich in den für die chemische Technik typischen Reaktionsapparaten durchgeführt werden. Bewährt haben sich Rührkessel, die mit einer Heizvorrichtung, sowie einer Einrichtung zum Zuführen des Azeotropbildners, z.B. einem Tauchrohr, ausgestattet sind.

In den folgenden Beispielen werden erfindungsgemäße Verfahren näher erläutert, es ist jedoch nicht auf die beschriebene Ausführungsform beschränkt.

### Beispiel 1: Herstellung von Tetraethylenglykol-di-3-methylbutyrat

Die Veresterung von Tetraethylenglykol mit 3-Methylbuttersäure wird in einem beheizbaren 2 I-Vierhalskolben durchgeführt, der mit einem Rührer, Innenthermometer und Tauchrohr ausgestattet ist und über eine Destillationsbrücke mit einer 1 1-Vorlage mit unterem Ablaß in Verbindung steht. In der Vorlage befindet sich ein Tauchrohr, das mittels Schlauch über eine Pumpe mit dem Tauchrohr des Reaktionskolbens verbunden ist.

In den Kolben werden 582,6 g Tetraethylenglykol, 766,2 g 3-Methylbuttersäure und 4,1 g Kaliumhydrogensulfat vorgelegt und unter Rühren auf 180°C erhitzt. Nach Erreichen einer Innentemperatur von 160°C werden stündlich 700 ml Cyclohexan aus der Vorlage über das Tauchrohr in den Kolben gepumpt. Das gleichzeitig abdestillierende Cyclohexan/Wasser-Azeotrop wird in der Vorlage aufgefangen und getrennt. Die obere Phase bestehend aus überwiegend Cyclohexan mit geringen Anteilen Carbonsäure wird wieder in den Reaktor zurückgeführt.

Der Reaktionsverlauf wird durch kontinuierliche Auswaage des in der Vorlage anfallenden Wassers sowie durch Probenahme und gaschromatographische Untersuchung der Proben verfolgt. Die nachfolgende Tabelle zeigt die Ergebnisse der gaschromatographischen Untersuchung.:

| | 1 Std. | 2 Std. | 3 Std. | 5 Std. | 10Std. |
|---|---|---|---|---|---|
| Cyclohexan | 4,5 % | 2,9 % | 2,6 % | 4,4 % | 5,9 % |
| 3-Methylbuttersäure | 48,0 | 37,1 | 24,8 | 10,8 | 9,5 % |
| Tetraethylenglykol | 14,7 | 10,4 | 5,9 % | 0,2 % | |
| Monoester | 22,5 | 30,7 | 32,1 | 8,2 % | 0,5 % |
| Diester | 6,5 % | 17,0 | 32,1 | 71,2 | 75,5 |
| Sonstige | 3,8 % | 1,9 % | 2,5 % | 5,2 % | 8,6 % |

Der nach Abschluß der Veresterung vorliegende Rohester (1189,9g) wird durch einfache Filtration vom Katalysator befreit und destillativ gereinigt; der Siedepunkt beträgt 189°C/1 mbar. Bei einer Dichte von 1,020 g/cm³ bei 20°C beträgt die Erstarrungstemperatur von Tetraethylenglykol-di-3-methylbutyrat <-30°C, bei 20°C weist der Diester eine Viskosität von 16,8 mPa•s auf.

### Beispiel 2: Herstellung von Tetraethylenglykol-di-isononanoat

Analog der in Beispiel 1 beschriebenen Estersynthese werden 388,4 g Tetraethylenglykol und 791,2 g Isononansäure in Gegenwart von 2,8 g Kaliumhydrogensulfat umgesetzt. Nach 10-stündiger Reaktion bei 180°C fallen 1127,7 g Rohester mit folgender Zusammensetzung an:

| | |
|---|---|
| Cyclohexan | 5,9 % |
| Isononansäure | 12,5 % |
| Tetraethylenglykol | - |
| Monoester | 0,1 % |
| Diester | 77,4 % |
| Sonstige | 4,1 % |

Der Katalysator wird durch einfache Filtration abgetrennt und der Ester durch Destillation gereinigt; der Siedepunkt von Tetraethylenglykol-di-isononanoat beträgt 219°C/1 mbar.

### Beispiel 3: Herstellung von Tetraethylenglykol-di-2-methylbutyrat

Analog der in Beispiel 1 beschriebenen Estersynthese werden 582,6 g Tetraethylenglykol und 766,0 g 2-Methylbuttersäure in Gegenwart von 4,1 g Kaliumhydrogensulfat umgesetzt. Nach 10-stündiger Reaktion bei 180°C fallen 1200,4 g Rohester mit folgender Zusammensetzung an:

| | |
|---|---|
| Cyclohexan | 6,4 % |
| 2-Methylbuttersäure | 10,2 % |
| Tetraethylenglykol | - |
| Monoester - | 0,8 % |
| Diester | 75,2 % |
| Sonstige | 7,4 % |

Der Katalysator wird durch einfache Filtration abgetrennt und der Ester durch Destillation gereinigt; der Siedepunkt beträgt 186°C/1 mbar. Bei einer Dichte von 1,022 g/cm³ bei 20°C beträgt die Erstarrungstemperatur von Tetraethylenglykol-di-2-methylbutyrat <-30°C, bei 20°C weist der Diester eine Viskosität von 12,7 mPa•s auf.

### Beispiel 4: Herstellung von Tetraethylenglykol-di-n-pentanoat

Analog der in Beispiel 1 beschriebenen Estersynthese werden 582,6 g Tetraethylenglykol und 766,0 g n-Pentansäure in Gegenwart von 4,1 g Kaliumhydrogensulfat umgesetzt. Nach 5-stündiger Reaktion bei 180°C fallen 1225,2 g Rohester mit folgender Zusammensetzung an:

| | |
|---|---|
| Cyclohexan | 7,2 % |
| n-Pentansäure | 10,2 % |
| Tetraethylenglykol | - |
| Monoester | 0,2 % |
| Diester | 76,7 % |
| Sonstige | 5,7 % |

Der Katalysator wird durch einfache Filtration abgetrennt und der Ester durch Destillation gereinigt; der Siedepunkt beträgt 196°C/1 mbar. Bei einer Dichte von 1,026 g/cm³ bei 20°C beträgt die Erstarrungstemperatur von Tetraethylenglykol-di-n-pentanoat -42°C, bei 20°C weist der Diester eine Viskosität von 14,0 mPa•s auf.

### Beispiel 5: Herstellung von Tetraethylenglykol-di-isobutyrat

Analog der in Beispiel 1 beschriebenen Estersynthese werden 621,4 g Tetraethylenglykol und 704,8 g Isobuttersäure in Gegenwart von 4,4 g Kaliumhydrogensulfat umgesetzt. Nach 10-stündiger Reaktion bei 180°C fallen 1201,0 g Rohester mit folgender Zusammensetzung an:

| | |
|---|---|
| Cyclohexan | 12,2 % |
| Isobuttersäure | 11,3 % |
| Tetraethylenglykol | - |
| Monoester | 2,3 % |
| Diester | 71,3 % |
| Sonstige | 2,9.%. |

Der Katalysator wird durch einfache Filtration abgetrennt und der Ester durch Destillation gereinigt; der Siedepunkt beträgt 171 °C/1,4 mbar.

### Beispiel 6: Herstellung von Triethylenglykol-di-isononanoat

Analog der in Beispiel 1 beschriebenen Estersynthese werden 375,5 g Triethylenglykol und 988,8 g Isononansäure in Gegenwart von 3,4 g Kaliumhydrogensulfat umgesetzt. Nach 6-stündiger Reaktion bei 180°C fallen 1281,6 g Rohester mit folgender Zusammensetzung an:

| | |
|---|---|
| Cyclohexan | 6,4 % |
| Isononansäure | 17,5 % |
| Triethylenglykol | - |
| Monoester | - |
| Diester | 69,4 % |
| Sonstige | 6,7 % |

Der Katalysator wird durch einfache Filtration abgetrennt und der Ester durch Destillation gereinigt; der Siedepunkt beträgt 220°C/0,8 mbar.

### Beispiel 7: Herstellung von Tetraethylenglykol-di-n-hexanoat

Analog der in Beispiel 1 beschriebenen Estersynthese werden 485,5 g Tetraethylenglykol und 726,3 g n-Hexansäure in Gegenwart von 3,4 g Kaliumhydrogensulfat umgesetzt. Nach 5-stündiger Reaktion bei 180°C fallen 1122,1 g Rohester mit folgender Zusammensetzung an:

| | |
|---|---|
| Cyclohexan | 7,4 % |
| n-Hexansäure | 12,4 % |
| Tetraethylenglykol | - |
| Monoester | 0,5 % |
| Diester | 75,9 % |
| Sonstige | 3,8 % |

Der Katalysator wird durch einfache Filtration abgetrennt und der Ester durch Destillation gereinigt; der Siedepunkt beträgt 205°C/0,7 mbar.

### Beispiel 8: Herstellung von Triethylenglykol-di-2-methylbutyrat

Analog der in Beispiel 1 beschriebenen Estersynthese werden 450,6 g Triethylenglykol und 766,0 g 2-Methylbuttersäure in Gegenwart von 4,1 g Kaliumhydrogensulfat umgesetzt. Nach 9-stündiger Reaktion bei 180°C fallen 1057,0 g Rohester mit folgender Zusammensetzung an:

| | |
|---|---|
| Cyclohexan | 6,1 % |
| 2-Methylbuttersäure | 9,3 % |
| Triethylenglykol | - |
| Monoester | 0,1 % |
| Diester | 77,7 % |
| Sonstige | 6,8 % |

Der Katalysator wird durch einfache Filtration abgetrennt und der Ester durch Destillation gereinigt; der Siedepunkt beträgt 150°C/1 mbar.

### Beispiel 9: Herstellung von Tetraethylenglykol-di-cyclohexancarboxylat

Analog der in Beispiel 1 beschriebenen Estersynthese werden 485,5 g Tetraethylenglykol und 801,3 g Cyclohexancarbonsäure in Gegenwart von 3,4 g Kaliumhydrogensulfat umgesetzt. Nach 5-stündiger Reaktion bei 180°C fallen 1215,7 g Rohester mit folgender Zusammensetzung an:

| | |
|---|---|
| Cyclohexan | 4,1 % |
| Cyclohexancarbonsäure | 11,3 % |
| Tetraethylenglykol | - |
| Monoester | 0,3 % |
| Diester | 79,3 % |
| Sonstige | 5,0 % |

Der Katalysator wird durch einfache Filtration abgetrennt und der Ester durch Destillation gereinigt.

### Beispiel 10: Herstellung von Triethylenglykol-di-cyclohexancarboxylat

Analog der in Beispiel 1 beschriebenen Estersynthese werden 375,5 g Triethylenglykol/und 801,3 g Cyclohexancarbonsäure in Gegenwart von 3,4 g Kaliumhydrogensulfat umgesetzt. Nach 7-stündiger Reaktion bei 180°C fallen 1080,3 g Rohester mit folgender Zusammensetzung an:

| | |
|---|---|
| Cyclohexan | 4,6 % |
| Cyclohexancarbonsäure | 11,5 % |
| Triethylenglykol | - |
| Monoester | 0,1 % |
| Diester | 78,4 % |
| Sonstige | 1,9 % |

Der Katalysator wird durch einfache Filtration abgetrennt und der Ester durch Destillation gereinigt.

### Beispiel 11: Herstellung von Tetraethylenglykol-di-2-methylhexanoat

Analog der in Beispiel 1 beschriebenen Estersynthese werden 485,5 g Tetraethylenglykol und 813,8 g 2-Methylhexansäure in Gegenwart von 3,4 g Kaliumhydrogensulfat umgesetzt. Nach 10-stündiger Reaktion bei 180°C fallen 1209,7 g Rohester mit folgender Zusammensetzung an:

| | |
|---|---|
| Cyclohexan | 5,9 % |
| 2-Methylhexansäure | 12,9 % |
| Tetraethylenglykol | - |
| Monoester | 0,1 % |
| Diester | 73,9% |
| Sonstige | 7,2% |

Der Katalysator wird durch einfache Filtration abgetrennt und der Ester durch Destillation gereinigt.

### Beispiel 12: Herstellung von Diethylenglykol-di-3-methylbutyrat

Analog der in Beispiel 1 beschriebenen Estersynthese werden 371,4 g Diethylenglykol und 893,4 g 3-Methylbuttersäure in Gegenwart von 4,8 g Kaliumhydrogensulfat umgesetzt. Nach 10-stündiger Reaktion bei 180°C fallen 1039,3 g Rohester mit folgender Zusammensetzung an:

| | |
|---|---|
| Cyclohexan | 5,2 % |
| 3-Methylbuttersäure | 12,7 % |
| Diethylenglykol | - |
| Monoester | 2,2 % |
| Diester | 78,8 % |
| Sonstige | 1,1 % |

Der Katalysator wird durch einfache Filtration abgetrennt und der Ester durch Destillation gereinigt.

## Patentansprüche

1. Verfahren zur Herstellung von Esterweichmachern durch Umsetzung von Mono-, Di-, Tri- oder Tetraethylenglykolen mit linearen oder verzweigten aliphatischen Monocarbonsäuren mit 3 bis 20 Kohlenstoffatomen in Gegenwart eines Schleppmittels zur Entfernung des im Verlauf der Umsetzung gebildeten Wassers als azeotropes Gemisch **dadurch** gekenneichnet, daß als Schleppmittel Substanzen aus der bougge bestehend aus Hexan, 1-Hexen, Cyclohexan und Toluol verwendet werden und daß das Schleppmittel dem Reaktionsgemisch bei Erreichen einer Temperatur von mindestens 140°C, insbesondere 150 bis 170°C, hinzugefügt wird.

2. Verfahren nach Anspruch 1 **dadurch gekennzeichnet, daß** als Schleppmittel Cyclohexan verwendet wird.

3. Verfahren nach Anspruch 1 oder 2 **dadurch gekennzeichnet, daß** das Schleppmittel dem Reaktionsgemisch in mehreren Anteilen zugesetzt wird.

4. Verfahren Anspruch 1 oder 2 **dadurch gekennzeichnet, daß** das Schleppmittel dem Reaktionsgemisch kontinuierlich zugesetzt wird.

5. Verfahren nach einem oder mehreren der Ansprüche 1 bis 4 **dadurch gekennzeichnet, daß** man die Monocarbonsäure in einem stöchiometrischen Überschuß von 0,05 bis 1,5 mol je mol Mono-, Di-, Tri- oder Tetraethylenglykol einsetzt.

6. Verfahren nach einem oder mehreren der Ansprüche 1 bis 5 **dadurch gekennzeichnet, daß** die Umsetzung in Gegenwart von Alkali- oder Erdalkalihydrogensulfat als Katalysator durchgeführt wird.

7. Verfahren nach einem oder mehreren der Ansprüche 1 bis 6 **dadurch gekennzeichnet, daß** man den Katalysator in einer Menge von 0,01 bis 1,0 Gew.-%, bezogen auf das Reaktionsgemisch, verwendet.

8. Verfahren nach einem oder mehreren der Ansprüche 1 bis 7 **dadurch gekennzeichnet, daß** man die Umsetzung bei Temperaturen von 150 bis 250°C durchführt.

## Claims

1. Process for preparing ester plasticizers by reacting monoethylene, diethylene, triethylene or tetraethylene glycols with linear or branched aliphatic monocarboxylic acids having from 3 to 20 carbon atoms in the presence of an entrainer for removal of the water formed during the reaction as an azeotropic mixture, **characterized in that** the entrainer used is a substance from the group consisting of hexane, 1-hexene, cyclohexane and toluene and **in that** the entrainer is added to the reaction mixture after a temperature of at least 140°C, in particular from 150 to 170°C, has been reached.

2. Process according to Claim 1, **characterized in that** the entrainer used is cyclohexane.

3. Process according to Claim 1 or 2, **characterized in that** the entrainer is added to the reaction mixture in a plurality of portions.

4. Process according to Claim 1 or 2, **characterized in that** the entrainer is added continuously to the reaction mixture.

5. Process according to one or more of Claims 1 to 4, **characterized in that** the monocarboxylic acid is used in a stoichiometric excess of from 0.05 to 1.5 mol per mole of monethylene, diethylene, triethylene or tetraethylene glycol.

6. Process according to one or more of Claims 1 to 5, **characterized in that** the reaction is carried out in the presence of an alkali metal hydrogensulfate or alkaline earth metal hydrogensulfate as catalyst.

7. Process according to one or more of Claims 1 to 6, **characterized in that** the catalyst is used in an amount of from 0.01 to 1.0% by weight, based on the reaction mixture.

8. Process according to one or more of Claims 1 to 7, **characterized in that** the reaction is carried out at temperatures of from 150 to 250°C.

## Revendications

1. Procédé en vue de la fabrication de plastifiants d'esters par réaction de mono-, di-, tri- ou tétra-éthylèneglycols avec des acides monocarboxyliques aliphatiques, linéaires ou ramifiés, ayant de 3 à 20 atomes de carbone, en présence d'un agent entraînant, en vue de l'élimination de l'eau formée au cours de la réaction en tant que mélange azéotrope, **caractérisé en ce que** l'on utilise, en tant qu'agent entraînant, des susbtances provenant du groupe se composant de l'hexane, du 1-hexène, du cyclohexane et du toluène, et **en ce que** l'on ajoute l'agent entraînant au mélange réactionnel lorsque l'on parvient à une température d'au moins 140 °C, en particulier de 150 à 170 °C.

2. Procédé selon la revendication 1, **caractérisé en ce que** l'on utilise, en tant qu'agent entraînant, le cyclohexane.

3. Procédé selon la revendication 1 ou la revendication 2, **caractérisé en ce que** l'agent entraînant est ajouté au mélange réactionnel en plusieurs portions.

4. Procédé selon la revendication 1 ou la revendication 2, **caractérisé en ce que** l'agent entraînant est ajouté au mélange réactionnel en continu.

5. Procédé selon l'une quelconque des revendications 1 à 4, **caractérisé en ce que** l'on utilise l'acide monocarboxylique dans un excès stoechiométrique de 0,05 à 1,5 moles par mole de mono-, di-, tri ou tétra- éthylèneglycol.

6. Procédé selon l'une quelconque des revendications 1 à 5, **caractérisé en ce que** la réaction est effectuée en présence d'hydrogénosulfate de métaux alcalins ou de métaux alcalinoterreux en tant que catalyseur.

7. Procédé selon l'une quelconque des revendications 1 à 6, **caractérisé en ce que** l'on utilise le catalyseur dans une quantité de 0,01 à 1,0% en poids, par rapport au mélange réactionnel.

8. Procédé selon l'une quelconque des revendications 1 à 7, **caractérisé en ce que** l'on effectue la réaction à des températures de 150 à 250 °C.
